# EUROPÄISCHE PATENTSCHRIFT

(11) **EP 0 910 581 B1**
(45) Veröffentlichungstag und Bekanntmachung des Hinweises auf die Patenterteilung: **05.04.2000**
(21) Anmeldenummer: 96923903.7
(22) Anmeldetag: 24.06.1996
(51) Int. Cl.: C07K 14/755

(54) **VERFAHREN ZUR AFFINITÄTSCHROMATOGRAPHISCHEN AUFREINIGUNG VON FAKTOR VIII**
METHOD FOR THE AFFINITY-CHROMATOGRAPHIC PURIFICATION OF FACTOR VIII
PROCEDE POUR LA PURIFICATION PAR CHROMATOGRAPHIE D'AFFINITE DU FACTEUR VIII

(43) Veröffentlichungstag der Anmeldung: 28.04.1999
(73) Patentinhaber: Max-Planck-Institut für Physiologische und Klinische Forschung Kerckhoff-Klink GmbH, 61231 Bad Neuheim (DE)
(72) Erfinder: MÜLLER-BERGHAUS, Gert, D-61239 Ober-Mörlen (DE); PÖTZSCH, Bernd, D-35390 Gie en (DE); SCHWINN, Horst, D-55129 Mainz (DE)
(74) Vertreter: Weiss, Wolfgang, Dipl.-Chem. Dr.
(86) Internationale Anmeldenummer: EP9602746
(87) Internationale Veröffentlichungsnummer: WO9749730

(56) Entgegenhaltungen:
- EP-A- 0 295 645
- DE-A- 19 521 313
- BIOLOGICAL ABSTRACTS, vol. 74, 1982 Philadelphia, PA, US; abstract no. 44256, XP002026789 & BLOOD, Bd. 59, Nr. 3, 1982, Seiten 615-624, G J KNUTSON & D N FASS: "Porcine factor VIII C prepared by affinity interaction with von Willebrand factor and heterologous antibodies sodium dodecyl sulfate poly acrylamide gel"

## Beschreibung

Die vorliegende Erfindung betrifft ein Verfahren zur affinitätschromatographischen Aufreinigung von Faktor VIII durch Adsorption an immobilisierten von Willebrand-Faktor.

Die Reinigung von humanem Faktor VIII (FVIII) aus biologischen Flüssigkeiten wie etwa Plasma, Plasmafraktionen oder Plasmaderivaten unter Verwendung affinitätschromatographischer Methoden ist seit langem bekannt.

So beschreiben Tuddenham et al (J. Lab. Clin. Med. 93 (1979), 40-53) ein solches Reinigungsverfahren unter Verwendung von immobilisierten polyklonalen Antikörpern, die aus dem Plasma von Inhibitorpatienten gewonnen wurden.

Veerman E.C.I. et al (Thromb. Res. 33 (1983), 89-93) beschreiben ein Aufreinigungsverfahren unter Verwendung monoklonaler Antikörper gegen humanen FVIII.

Weiterhin sind Verfahren zur Aufreinigung von FVIII unter Verwendung immobilisierter monoklonaler muriner Antikörper gegen humanen von Willebrand-Faktor (vWF) bekannt, bei dem vWF/FVIII-Komplexe adsorbiert und anschließend vWF-freier FVIII nach bekannten Methoden isoliert wird (vgl. z.B. EP-A-0083 438 und US-A-4,831,118).

Ein Nachteil dieser Verfahren ist jedoch, daß sich eine Trennung des FVIII von seinem natürlichen Stabilisator, dem vWF, nachteilig auf die Stabilität von FVIII auswirkt. Weiterhin ist es für bestimmte Therapieformen ungünstig, eine vollständig vWF-freie FVIII-Präparation zu erhalten (Hornsey et al, Thromb. Haemost. 57 (1987), 102-105).

Eine Adsorption von FVIII an immobilisierten plasmatischen vWF ist bereits bekannt (vgl. Leyte et al, Biochem. J. 257 (1989), 679-683). Dabei ergab sich jedoch, daß nur etwa 1-2 % der plasmatischen FVIII-Aktivität an immobilisierten plasmatischen vWF gebunden werden. Ein wirtschaftlich arbeitendes Verfahren ist mit einem solchen Ergebnis nicht vorstellbar.

Eine Aufgabe der vorliegenden Erfindung bestand somit darin, die Nachteile des Standes der Technik mindestens teilweise zu beseitigen. Insbesondere soll ein Verfahren bereitgestellt werden, mit dem auf einfache und kostengünstige Weise eine hohe Reinigungseffizienz erzielt werden kann.

Diese Aufgabe wird gelöst durch ein Verfahren zur affinitätschromatographischen Aufreinigung von Faktor VIII, dadurch gekennzeichnet, daß man eine Faktor VIII enthaltende biologische Flüssigkeit mit immobilisiertem zellulärem von Willebrand-Faktor oder einem Derivat davon unter solchen Bedingungen in Kontakt bringt, daß eine Adsorption von Faktor VIII an den immobilisierten von Willebrand-Faktor oder das Derivat davon erfolgt, Verunreinigungen abtrennt und adsorbierten Faktor VIII eluiert. Überraschenderweise wurde gefunden, daß zellulärer, z.B. durch Endothel-Zellen gebildeter vWF im Gegensatz zu plasmatischem vWF nach Immobilisierung hervorragend für die Aufreinigung von FVIII geeignet ist. Ein von Endothel-Zellen produzierter vWF (EC-vWF) besitzt nämlich eine wesentlich höhere Affinität zu FVIII als plasmatischer vWF, so daß eine Gewinnung der FVIII-Aktivität aus biologischen Flüssigkeiten mit hoher Reinheit und Ausbeute möglich ist.

Zellulärer vWF wird vorzugsweise aus kultivierten humanen Zellen, vorzugsweise aus kultivierten humanen Endothel-Zellen, z.B. aus humanen Endothel-Zellen der Nabelschnurvene (HUVEC) gewonnen, an eine Festphase gebunden und mit einer biologischen Flüssigkeit inkubiert. Nach Abtrennung von Verunreinigungen (nichtbindende oder unspezifisch bindende Verunreinigungen) wird der adsorbierte FVIII vorzugsweise durch Anlegen eines Salzgradienten eluiert. Die Vorteile des erfindungsgemäßen Verfahren bestehen insbesondere in der ausschließlichen Verwendung von humanem Material, einer wenig zeitintensiven Reinigung und einer geringen Proteolyserate des gereinigten FVIII. Dies führt zu einer außergewöhnlich hohen Spezifität des gereinigten Materials.

Als biologische Flüssigkeiten werden vorzugsweise Humanplasma, Plasmafraktionen, Plasmaderivate, z.B. Cryopräzipitate, oder Kulturüberstände von FVIII produzierenden Zellen verwendet. Gegebenenfalls kann die biologische Flüssigkeit vorab einer chromatographischen Reinigung, z.B. einer Aluminiumhydroxidadsorption unterzogen werden.

Bei Verwendung von Plasma als biologischer Flüssigkeit ist eine Vorverdünnung von ca. 1:2 und der Zusatz von Proteinaseinhibitoren bevorzugt.

Weiterhin ist bevorzugt, die FVIII-enthaltende biologische Flüssigkeit für eine Dauer von mindestens 30 min. mit dem immobilisierten vWF in Kontakt zu halten. Besonders bevorzugt ist eine Kontaktdauer von mindestens 1 h. Die Inkubationstemperatur ist vorzugsweise 10 bis 37 °C.

Als immobilisierter vWF kann monomerer vWF oder Derivate davon, gewählt aus dimerer, oligomerer oder multimerer vWF verwendet werden.

Der vWF oder das Derivat davon wird an ein inertes, vorzugsweise teilchenförmiges Trägermaterial immobilisiert. Beispiele für geeignete Trägermaterialien sind anorganische Träger wie Kieselgel, Silikate, poröse Gläser oder organische Trägermaterialien wie etwa ggf. vernetzte Polysaccharide, z.B. Cellulose, Cellulosederivate, Dextrane oder ggf. modifizierte Agarose. Ein bevorzugter Träger ist Sepharose®, ein Material auf Basis von modifizierter Agarose, dessen Polysaccharidketten zu einem dreidimensionalen Netzwerk verknüpft sind.

Nach der Adsorption von FVIII aus der biologischen Flüssigkeit an den immobilisierten vWF wird die biologische Flüssigkeit abgetrennt. Anschließend erfolgt vor der Elution vorzugsweise noch ein Waschvorgang, um unspezifisch an den Träger gebundene Komponenten, z.B. Plasmaproteine, zu entfernen.

Die nachfolgende Elution erfolgt vorzugsweise durch Erhöhung der Ionenstärke, z.B. durch Anlegen eines Gradienten mit zunehmender Salzkonzentration. Besonders geeignet hat sich ein NaCl-Gradient von 0,6-1,5 M erwiesen.

Die FVIII enthaltende biologische Flüssigkeit, die mit dem immobilisierten Träger in Kontakt gebracht wird, weist vorzugsweise einen pH-Wert von 6,4-7,2, besonders bevorzugt einen pH-Wert von ca. 6,8 auf. Weiterhin ist bevorzugt, daß der biologischen Flüssigkeit und ggf. der Waschlösung Hirudin zugesetzt wird.

Durch das erfindungsgemäße Verfahren ist es möglich, mindestens 50 % und besonders bevorzugt mindestens 70 % der FVIII-Aktivität aus der biologischen Flüssigkeit zu gewinnen. Weiterhin wird durch das erfindungsgemäße Verfahren eine FVIII-Präparation mit einer hohen Reinheit von vorzugsweise mindestens 50 IU/mg Protein, z.B. ca. 100 IU/mg-Protein erhalten.

Noch ein weiterer Vorteil des erfindungsgemäßen Verfahrens ist, daß man eine FVIII-Präparation gewinnt, die zusätzlich noch komplexierten vWF enthält. Vorzugsweise enthält die Präparation 1 U vWF pro 5-15 IU FVIII. Die Definition einer IU für FVIII und vWF findet sich bei Bloom et al., Haemostasis and Thrombosis, 3rd Edition, Churchill Livingstrone, Edinburgh, 1994.

Die durch das erfindungsgemäße Verfahren aufgereinigte vWF-FVIII enthaltende Präparation kann anschließend einem oder mehreren bekannten Verfahren zur Virusinaktivierung unterworfen werden (z.B. EP-A-0 131 740 und PCT/EP 94/00328).

Das erfindungsgemäße Verfahren wird weiterhin durch das nachfolgende Beispiel erläutert.

### BEISPIEL 1:

Es wird ein Verfahren beschrieben, das zur Reinigung von FVIII aus Humanplasma oder Plasmaderivaten geeignet ist. Grundlage der Methode ist eine unterschiedliche Bindungsaffinität von plasmatischen und endothelialem von vWF an FVIII. Endothelialer vWF (EC-vWF) wird aus kultivierten humanen Endothelzellen der Nabelschnurvene (HUVEC) gewonnen, an eine Festphase gebunden und mit Plasma inkubiert. Nach Entfernung von unspezifisch gebundenen Plasmaproteinen wird der gebundene FVIII mit einem Salzgradienten isoliert.

### 1.1 Gewinnung und Herstellung der EC-vWF-Matrix

EC-vWF wird aus dem Überstand von kultivierten HUVEC der zweiten und dritten Passage gewonnen. Dazu werden die Zellen in Kulturschalen mit einem Durchmesser von 150 mm kultivert, nach Erreichen der Konfluenz mit 37°C warmer Phosphat-gepufferter Salzlösung (PBS) gewaschen und anschließend mit PMA, einem Phorbolester, stimuliert. Diese Stimulierung führt zur Abgabe des intrazellulären vWF in den Kulturüberstand. Die gewonnenen Überstände werden vereinigt, der EC-vWF gereinigt und auf eine Endkonzentration von 2-5 mg/ml eingestellt. Nach Dialyse gegen einen Natriumhydrogencarbonatpuffer (0,1 M, pH 8,3) wird der EC-vWF an CNBr-aktivierte Sepharose 4B (Pharmacia) entsprechend den Angaben des Herstellers gekoppelt. Zur Absättigung unspezifischer Bindungsstellen wurde 1 M Glycin verwendet. Die EC-vWF-Sepharose kann in TBS, 0,01 % Thimerosal bis zur Verwendung gelagert werden.

### 1.2 Adsorption von FVIII an immobilisierten vWF

Als Ausgangsmaterial ist z.B. mit Natriumcitrat antikoaguliertes Plasma, aus diesem Plasma gewonnenes Cryopräzipitat oder ein anderes Plasmaderivat geeignet. Das Cryopräzipitat kann vorab einer Aluminiumhydroxidadsorption (2 % (vol/vol) Aluminiumhydroxid (Calbiochem, USA), 20 min Raumtemperatur, 10.000 g Zentrifugation) unterzogen und im Verhältnis 1:2 mit 50 ml Auftragspuffer (100mM NaCl, 50 mM Tris, 80 mM Calciumchlorid, 100 ng r-Hirudin, pH 6,8) verdünnt werden. Dann wird das Cryo/Puffergemisch mit 10.000 x g 30 min bei Raumtemperatur abzentrifugiert. Auch bei Verwendung von Plasma ist eine Vorverdünnung von 1:2 bevorzugt. Die Inkubation der vorbereitenden Probe mit der EC-vWF-Sepharose kann sowohl in einem geschlossenen Gefäß als auch in offener Chromatographieeinrichtung erfolgen. Vor Beginn der Elution wird die Säulenmatrix mit einem Zehnfachen des Säulenvolumens mit TBS (500 mM NaCl, 20 mM Tris-HCl, 50 ng r-Hirudin) gewaschen.

### 1.3 Elution des gebundenen FVIII

Die Elution erfolgt mit einem kontinuierlichen Gradienten von 0,6 - 1,5 M NaCl in 20 mM Tris-HCl, 150 mM MgCl₂, pH 6,8. FVIII enthaltende Proben werden vereinigt, gegen PBS, pH 6,8 dialysiert und vorzugsweise nach Zugabe von 0,1 % Humanserumalbumin (HSA) bei -80°C gelagert. Eine Lagerung ist ohne Aktivitätsverlust bis zu mindestens 6 Monaten möglich.

### 1.4 Beispiel einer typischen Reinigung

Ein kommerziell erhältliches Cryopräzipitat wurde bei 37°C aufgetaut, wie bereits beschrieben einer Aluminiumhydroxidadsorption unterzogen und mit Auftragspuffer im Verhältnis 1:2 verdünnt. Mit je 20 ml dieses Materials wurden drei voneinander unabhängige Reinigungen mit jeweils der gleichen Säule durchgeführt.

| Fraktion | Protein (mg) | vWF-Antigen (µg) | FVIII-Aktivität (IU) | FVIII-Ag (ng) | FVIII-Akt./ Protein-Ratio |
|---|---|---|---|---|---|
| Ausgangsmaterial | 27 | 284 | 117 | 783 | 4,3 |
| Eluat1 | 1,46 | 57 | 72 | 694 | 49 |
| Eluat2 | 0,91 | 47 | 89 | 673 | 97 |
| Eluat3 | 1,08 | 45 | 78 | 564 | 72,2 |

Nach jeder Reinigung wird die EC-vWF-Matrix zur Regeneration mit einer 1 M NaCl-Lösung (das fünffache Säulenvolumen) zur Entfernung von kontaminierenden Proteinen und anschließend mit TBS gewaschen. Eine Lagerung ist nach Zusatz von 0,01 % Thimerosal in diesem Puffer möglich. Vor Verwendung sollte die Matrix intensiv mit Auftragspuffer gewaschen werden.

## Patentansprüche

1. Verfahren zur affinitätschromatographischen Aufreinigung von Faktor VIII,
**dadurch gekennzeichnet**,
daß man eine Faktor VIII enthaltende biologische Flüssigkeit mit immobilisiertem zellulärem von Willebrand-Faktor oder einem Derivat davon gewählt aus Dimeren, Oligomeren oder Multimeren, unter solchen Bedingungen in Kontakt bringt, daß eine Adsorption von Faktor VIII an den immobilisierten von Willebrand-Faktor oder das Derivat davon erfolgt, Verunreinigungen abtrennt und adsorbierten Faktor VIII eluiert.

2. Verfahren nach Anspruch 1,
**dadurch gekennzeichnet,**
daß man als biologische Flüssigkeit Humanplasma, Plasmafraktionen, Plasmaderivate oder Kulturüberstände von Faktor VIII produzierenden Zellen verwendet.

3. Verfahren nach Anspruch 1,
**dadurch gekennzeichnet,**
daß man als biologische Flüssigkeit ein Human-Plasma-Cryopräzipitat verwendet.

4. Verfahren nach Anspruch 1-3,
**dadurch gekennzeichnet**,
daß man die biologische Flüssigkeit vorab einer chromatographischen Reinigung mit Aluminiumhydroxid unterzieht.

5. Verfahren nach einem der Ansprüche 1-4,
**dadurch gekennzeichnet**,
daß man immobilisierten von Willebrand-Faktor aus Endothelzellen oder ein Derivat davon verwendet.

6. Verfahren nach einem der Ansprüche 1-5,
**dadurch gekennzeichnet**,
daß der von Willebrand-Faktor an ein teilchenförmiges Trägermaterial immobilisiert ist.

7. Verfahren nach einem der Ansprüche 1-6,
**dadurch gekennzeichnet**,
daß das Trägermaterial ausgewählt wird aus Kieselgel, Silikate, porösen Gläsern und Polysacchariden.

8. Verfahren nach einem der Ansprüche 1-7,
**dadurch gekennzeichnet**,
daß die biologische Flüssigkeit für eine Dauer von mindestens 30 min. mit dem immobilisierten von Willebrand-Faktor in Kontakt gehalten wird.

9. Verfahren nach Anspruch 8,
**dadurch gekennzeichnet**,
daß die Kontaktdauer mindestens 1 h beträgt.

10. Verfahren nach einem der Ansprüche 1-9,
**dadurch gekennzeichnet**,
daß vor der Elution ein Waschvorgang erfolgt.

11. Verfahren nach einem der Ansprüche 1-10,
**dadurch gekennzeichnet,**
daß die Elution durch Erhöhung der Ionenstärke erfolgt.

12. Verfahren nach Anspruch 11,
**dadurch gekennzeichnet**,
daß ein Gradient von zunehmender Salzkonzentration angelegt wird.

13. Verfahren nach einem der Ansprüche 1-12,
**dadurch gekennzeichnet**,
daß die biologische Flüssigkeit einen pH-Wert von 6,4-7,2 aufweist.

14. Verfahren nach einem der Ansprüche 1-13,
**dadurch gekennzeichnet**,
daß man der biologischen Flüssigkeit Hirudin zusetzt.

15. Verfahren nach einem der Ansprüche 1-14,
**dadurch gekennzeichnet**,
daß man mindestens 50 % der Faktor VIII-Aktivität aus der biologischen Flüssigkeit gewinnt.

16. Verfahren nach einem der Ansprüche 1-15,
**dadurch gekennzeichnet**,
daß man eine Faktor VIII Präparation mit einer Reinheit von mindestens 50 IU/mg Protein gewinnt.

17. Verfahren nach einem der Ansprüche 1-16,
**dadurch gekennzeichnet**,
daß man eine Faktor VIII-Präparation gewinnt, die komplexierten von Willebrand-Faktor enthält.

18. Verfahren nach Anspruch 17,
**dadurch gekennzeichnet**,
daß die Präparation 1 U von Willebrand-Faktor pro 5-15 IU Faktor VIII enthält.

19. Verfahren nach einem der Ansprüche 1-18,
**dadurch gekennzeichnet**,
daß man die Faktor VIII-Präparation anschließend einer Virusinaktivierung unterzieht.

## Claims

1. Process for the purification of factor VIII by affinity chromatography,
**wherein**
a biological fluid containing factor VIII is contacted with immobilized cellular von Willebrand factor or a derivative thereof selected from dimers, oligomers or multimers under such conditions that factor VIII is adsorbed to the immobilized von Willebrand factor or to the derivative thereof, impurities are separated and adsorbed factor VIII is eluted.

2. Process as claimed in claim 1,
**wherein**
human plasma, plasma fractions, plasma derivatives or culture supernatants of cells producing factor VIII are used as the biological fluid.

3. Process as claimed in claim 1,
**wherein**
a human plasma cryoprecipitate is used as the biological fluid.

4. Process as claimed in claim 1-3,
**wherein**
the biological fluid is firstly subjected to a chromatographic purification with aluminium hydroxide.

5. Process as claimed in one of the claims 1-4,
**wherein**
immobilized von Willebrand factor from endothelial cells or a derivative thereof is used.

6. Process as claimed in one of the claims 1-5,
**wherein**
the von Willebrand factor is immobilized on a particulate carrier material.

7. Process as claimed in one of the claims 1-6,
**wherein**
the carrier material is selected from silica gel, silicates, porous glasses and polysaccharides.

8. Process as claimed in one of the claims 1-7,
**wherein**
the biological fluid is kept in contact with the immobilized von Willebrand factor for a period of at least 30 min.

9. Process as claimed in claim 8,
**wherein**
the contact period is at least 1 h.

10. Process as claimed in one of the claims 1-9,
**wherein**
a wash process is carried out before elution.

11. Process as claimed in one of the claims 1-10,
**wherein**
the elution is achieved by increasing the ionic strength.

12. Process as claimed in claim 11,
**wherein**
a gradient of increasing salt concentration is applied.

13. Process as claimed in one of the claims 1-12,
**wherein**
the biological fluid has a pH value of 6.4 - 7.2

14. Process as claimed in one of the claims 1-13,
**wherein**
hirudin is added to the biological fluid.

15. Process as claimed in one of the claims 1-14,
**wherein**
at least 50 % of the factor VIII activity is isolated from the biological fluid.

16. Process as claimed in one of the claims 1-15,
**wherein**
a factor VIII preparation is obtained with a purity of at least 50 IU/mg protein.

17. Process as claimed in one of the claims 1-16,
**wherein**
a factor VIII preparation is obtained which contains complexed von Willebrand factor.

18. Process as claimed in claim 17,
**wherein**
the preparation contains 1 U von Willebrand factor per 5-15 IU factor VIII.

19. Process as claimed in one of the claims 1-18,
**wherein**
the factor VIII preparation is subsequently subjected to a virus inactivation.

## Revendications

1. Procédé pour la purification par chromatographie d'affinité du facteur VIII,
caractérisé en ce que,
l'on met en contact un fluide biologique contenant un facteur VIII avec un facteur von Willebrand cellulaire mobilisé ou son dérivé dans des conditions telles qu'il se produit une adsorption du facteur VIII sur le facteur von Willebrand immobilisé ou son dérivé, et en ce que l'on sépare les impuretés et en ce que l'on élue le facteur VIII adsorbé.

2. Procédé selon la revendication 1,
caractérisé en ce que,
l'on utilise comme fluide biologique du plasma humain, des fragments de plasma, des dérivés de plasma ou des surnageants de culture de cellules produisant le facteur VIII.

3. Procédé selon la revendication 1,
caractérisé en ce que,
comme liquide biologique, on utilise un cryoprécipité de plasma humain.

4. Procédé selon les revendications 1-3,
caractérisé en ce que,
l'on soumet préalablement le liquide biologique à une purification chromatographique avec de l'hydroxyde d'aluminium.

5. Procédé selon l'une des revendications 1-4,
caractérisé en ce que,
l'on utilise le facteur von Willebrand immobilisé à partir de cellules endothéliales ou de leur dérivé.

6. Procédé selon l'une des revendications 1-5,
caractérisé en ce que,
le facteur von Willebrand est immobilisé sur un matériau support particulaire.

7. Procédé selon l'une des revendications 1-6,
caractérisé en ce que,
le matériau support est choisi à partir de gel de silice, de silicates, de verres poreux et de polysaccharides.

8. Procédé selon l'une des revendications 1-7,
caractérisé en ce que,
le fluide biologique est maintenu en contact avec le facteur von Willebrand immobilisé pendant une durée d'au moins 30 minutes.

9. Procédé selon la revendication 8,
caractérisé en ce que,
la durée du contact est d'au moins 1 heure.

10. Procédé selon l'une des revendications 1-9,
caractérisé en ce qu'avant l'élution, on procède à un lavage.

11. Procédé selon l'une des revendications 1-10,
caractérisé en ce que,
l'élution s'effectue après augmentation de la force ionique.

12. Procédé selon la revendication 11,
caractérisé en ce que,
l'on applique un gradient de concentration saline croissante.

13. Procédé selon l'une des revendications 1-12,
caractérisé en ce que,
le fluide biologique présente une valeur de pH de 6,4-7,2.

14. Procédé selon l'une des revendications 1-13,
caractérisé en ce que
l'on ajoute de l'hirudine au fluide biologique.

15. Procédé selon l'une des revendications 1-14,
caractérisé en ce que,
l'on obtient au moins 50% de l'activité de facteur VIII à partir du fluide biologique.

16. Procédé selon l'une des revendications 1-15,
caractérisé en ce que,
l'on obtient une préparation de facteur VIII avec une pureté d'au moins 50 UI/mg de protéine.

17. Procédé selon l'une des revendications 1-16,
caractérisé en ce que,
l'on obtient une préparation de facteur VIII qui contient le facteur von Willebrand complexé.

18. Procédé selon la revendication 17,
caractérisé en ce que,
la préparation contient un 1U de facteur von Willebrand par 5-15 UI de facteur VIII.

19. Procédé selon l'une des revendications 1-18,
caractérisé en ce que,
l'on soumet consécutivement la préparation de facteur VIII à une inactivation virale.
